# EUROPEAN PATENT APPLICATION

(11) **EP 4 748 307 A1**
(43) Date of publication of application: **27.05.2026**
(21) Application number: 24842927.6
(22) Date of filing: 01.07.2024
(51) Int. Cl.: A61B 5/145, G01N 27/02, G01R 27/04

(54) **MEASUREMENT DEVICE**

(30) Priority: 20.07.2023 JP 2023118087
(71) Applicant: Sony Semiconductor Solutions Corporation, Atsugi-shi, Kanagawa 243-0014 (JP)
(72) Inventor: KONDO, Fumitaka, Atsugi-shi, Kanagawa 243-0014 (JP); KIMURA, Yuki, Atsugi-shi, Kanagawa 243-0014 (JP); MARUYAMA, Hirofumi, Atsugi-shi, Kanagawa 243-0014 (JP)
(74) Representative: Müller Hoffmann & Partner
(86) International application number: PCT/JP2024/023725
(87) International publication number: WO 2025/018133

(57) **Abstract**

The present technology relates to a measurement apparatus capable of improving measurement accuracy.

The measurement apparatus includes: a circuit portion that is arranged on one surface of a sensor substrate and measures an S parameter; and a sensor electrode that is arranged on the other surface of the sensor substrate and irradiates a measurement target with a signal for measuring the S parameter output from the circuit portion, in which a distance between the circuit portion and the sensor electrode is equal to or less than a predetermined distance in plan view, and a terminal that outputs the signal of the circuit portion is directly connected to the sensor substrate. The present technology can be applied to a glucose sensing system.

## Description

### TECHNICAL FIELD

The present technology relates to a measurement apparatus, and particularly to a measurement apparatus capable of improving measurement accuracy.

### BACKGROUND ART

In related art, a non-invasive biological information sensor that measures biological information such as a glucose level (blood glucose level) using an infrared laser, a microwave, a millimeter wave, or the like, has been studied and commercialized.

As such a biological information sensor, for example, a measurement apparatus that measures a glucose level by measuring an S parameter is known.

In addition, for example, there is also proposed a component concentration measurement apparatus that irradiates a measurement target with an electromagnetic wave to measure a complex dielectric constant, acquires a dielectric spectrum, measures a temperature of the measurement target, and corrects the dielectric spectrum according to the measured temperature (see, for example, Patent Document 1).

### CITATION LIST

### PATENT DOCUMENT

Patent Document 1: Japanese Patent Application Laid-Open No. 2019-190895

### SUMMARY OF THE INVENTION

### PROBLEMS TO BE SOLVED BY THE INVENTION

Meanwhile, in a measurement apparatus that measures a glucose level, or the like, it is desired to improve measurement accuracy.

The present technology has been made in view of such a situation, and an object thereof is to improve measurement accuracy.

### SOLUTIONS TO PROBLEMS

A measurement apparatus according to one aspect of the present technology includes: a circuit portion that is arranged on one surface of a sensor substrate and measures an S parameter; and a sensor electrode that is arranged on the other surface of the sensor substrate and irradiates a measurement target with a signal for measuring the S parameter output from the circuit portion, in which a distance between the circuit portion and the sensor electrode is equal to or less than a predetermined distance in plan view, and a terminal that outputs the signal of the circuit portion is directly connected to the sensor substrate.

In one aspect of the present technology, a measurement apparatus includes: a circuit portion that is arranged on one surface of a sensor substrate and measures an S parameter; and a sensor electrode that is arranged on the other surface of the sensor substrate and irradiates a measurement target with a signal for measuring the S parameter output from the circuit portion, in which a distance between the circuit portion and the sensor electrode is equal to or less than a predetermined distance in plan view, and a terminal that outputs the signal of the circuit portion is directly connected to the sensor substrate.

### BRIEF DESCRIPTION OF DRAWINGS

Fig. 1 is a view illustrating a configuration example of appearance of a smartwatch.
Fig. 2 is a view illustrating a configuration example of inside of a main body of the smartwatch.
Fig. 3 is a flowchart for explaining measurement processing.
Fig. 4 is a view for explaining a positional relationship between a VNA chip and a sensor electrode.
Fig. 5 is a view for explaining the positional relationship between the VNA chip and the sensor electrode.
Fig. 6 is a view for explaining determination of normality of a glucose level.
Fig. 7 is a view for explaining use of body temperature detection.
Fig. 8 is a view for explaining heat dissipation of the VNA chip.
Fig. 9 is a view for explaining correction of an S parameter.
Fig. 10 is a view for explaining setting of an average number of times.
Fig. 11 is a view for explaining the setting of the average number of times.
Fig. 12 is a view for explaining the setting of the average number of times.
Fig. 13 is a view illustrating a configuration example of a measurement apparatus.
Fig. 14 is a flowchart indicating measurement processing.
Fig. 15 is a view for explaining the setting of the average number of times.
Fig. 16 is a view for explaining the setting of the average number of times.

### MODE FOR CARRYING OUT THE INVENTION

Embodiments to which the present technology is applied will be described below with reference to the drawings.

### <First Embodiment>

### <Configuration Example of Smartwatch>

The present technology relates to a vector network analyzer (VNA) module including a VNA chip and a sensor system equipped with the VNA module.

For example, the VNA module is a highly versatile device capable of performing antenna measurement and dielectric constant measurement of a substance in addition to impedance measurement and gain and loss measurement of a high frequency device by transmitting a signal and measuring an amplitude and a phase of a reflected wave and a transmitted wave of the signal.

The present technology can be applied to a sensor system such as a biological information sensor or a soil moisture sensor using the VNA module.

Hereinafter, as a specific embodiment, a case where the present technology is mainly applied to a glucose sensor system that measures a glucose level as biological information of a wearer will be described as an example.

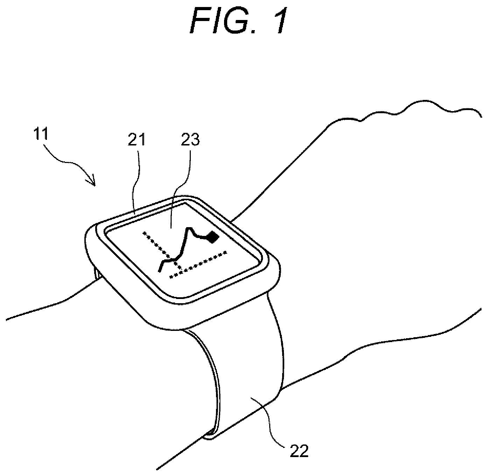

Fig. 1 is a view illustrating a configuration example of appearance of an embodiment of a smartwatch to which the present technology is applied.

A smartwatch 11 illustrated in Fig. 1 functions as a glucose sensor system which is an example of a biological information sensor to which the present technology is applied. In particular, the smartwatch 11 functions as a non-invasive measurement apparatus that measures a glucose level (blood glucose level) by measuring an S parameter related to the wearer.

The smartwatch 11 can be worn on an arm portion of the user, and includes a main body 21 and a band portion 22. The main body 21 is fixed to the arm portion of the user by the band portion 22 which is a mounting tool.

The smartwatch 11 measures a glucose level and a body temperature of the user who is the wearer. In addition, the main body 21 is provided with a small display 23 so that various types of information such as a glucose level and a body temperature obtained as a measurement result can be displayed on the display 23.

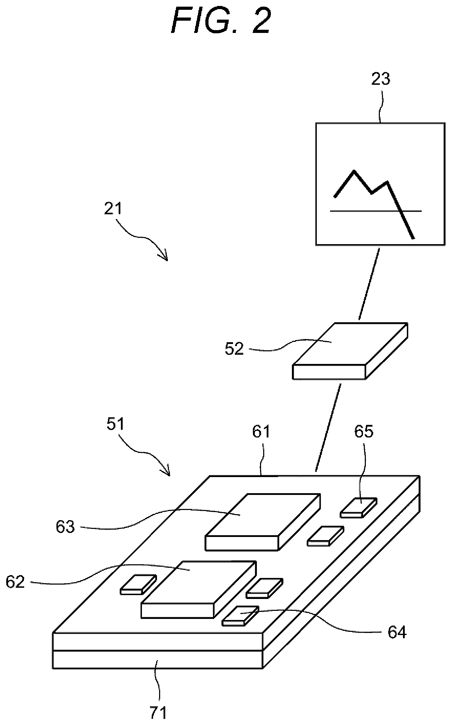

As illustrated in Fig. 2, a glucose sensing module 51 and a main central processing unit (CPU) 52 are provided inside the main body 21 of the smartwatch 11. Further, the display 23 is also connected to the main CPU 52. Furthermore, the main body 21 also includes a communication unit (not illustrated) and a battery therein. The communication unit includes, for example, a wireless module in which a Wi-Fi function (registered trademark) or a Bluetooth function (registered trademark) is incorporated, and exchanges (transmits and receives) data with an external terminal. In particular, the communication unit is arranged on a side opposite to a side of the sensor substrate 61 (side of the arm of the user) of the glucose sensing module 51, that is, on an upper side in the drawing such that the glucose sensing module 51 does not interfere with communication by the communication unit. In addition, the communication unit and the glucose sensing module 51 are arranged so as to overlap with each other in plan view, that is, when viewed from a direction perpendicular to a sensor substrate 61, thereby achieving downsizing of the smartwatch 11.

The glucose sensing module 51 is a VNA module provided with a VNA chip (VNA integrated circuit).

The glucose sensing module 51 includes the sensor substrate 61 including a semiconductor substrate, or the like. Components from a VNA chip 62 to a flash memory 65 are provided on one surface of the sensor substrate 61, and a sensor electrode described later is provided on the other surface of the sensor substrate 61.

Specifically, the VNA chip 62, a microcomputer 63, a temperature sensor 64, and the flash memory 65 are provided on the surface of the sensor substrate 61 on the side of the arm of the user, that is, on the surface opposite to a surface on a side in contact with the arm of the user when the smartwatch 11 is worn.

Hereinafter, the surface of the sensor substrate 61 on the side opposite to side of the arm of the user, that is, the surface (upper surface) on the side on which the components from the VNA chip 62 to the flash memory 65 are provided will be also referred to as a front surface, and the surface (lower surface) on the side opposite to the side of the front surface of the sensor substrate 61 will be also referred to as a back surface.

The VNA chip 62 is a VNA integrated circuit that irradiates the arm portion of the user to be measured with an electromagnetic wave in a microwave or millimeter wave band, that is, a radio frequency (RF) signal as an incident signal via a probe (not illustrated), or the like, to measure an S parameter. In other words, the VNA chip 62 functions as a circuit portion that measures the S parameter.

More specifically, a tissue around the arm portion, which is a portion of a living body, specifically, a body tissue including keratin, skin cells, blood vessels (blood), and the like, is set as a measurement target. Note that the portion of the user to be measured is not limited to the arm portion, and may be any portion such as an earlobe, a palm, a foot, or an abdomen. Further, the measurement target is not limited to a human, and may be an animal. In the present technology, by setting a living body as the measurement target, not only measurement can be performed more easily than in a case where the measurement target is a liquid in a container, or the like, but also biological information of the measurement target can be acquired in real time.

For example, the back surface of the sensor substrate 61 is a sensor electrode area 71 provided with one or a plurality of sensor electrodes functioning as probes. Each sensor electrode is connected to a port of the VNA chip 62.

The VNA chip 62 irradiates the arm portion of the user to be measured with an incident signal for measuring the S parameter from a predetermined port (hereinafter, referred to as a port 1) via the sensor electrode, and receives the incident signal reflected inside the arm of the user and returned via the port 1 as a reflected signal.

Furthermore, for example, in a case where another port 2 different from the port 1 is also provided in the VNA chip 62, the VNA chip 62 receives, as a transmitted signal, the incident signal transmitted through the arm portion of the user to be measured. In this case, the incident signal is applied to the measurement target via the port 1, and a transmitted signal corresponding to the incident signal is received via the sensor electrode and the port 2.

The VNA chip 62 calculates the S parameter on the basis of at least the incident signal and the reflected signal among the incident signal, the reflected signal, and the transmitted signal. In other words, at the time of measuring biological information such as a glucose level, the VNA chip 62 measures the S parameter.

The S parameter is a parameter related to a magnitude (amplitude) and a phase of an RF signal such as the reflected signal and the transmitted signal. For example, in a case where only the port 1 is used for measurement, S11 is calculated as the S parameter on the basis of the incident signal and the reflected signal. S11 represents change in magnitude and phase of the reflected signal with respect to the incident signal incident on the measurement target.

In addition, for example, in a case where the port 1 and the port 2 are used for measurement, S11 and S21 are calculated as the S parameter on the basis of the incident signal, the reflected signal, and the transmitted signal. S21 represents change in magnitude and phase of the transmitted signal with respect to the incident signal incident on the measurement target.

The microcomputer 63 controls operation of the VNA chip 62 and calculates biological information such as a glucose level on the basis of the S parameter obtained by the VNA chip 62.

The temperature sensor 64 is provided in proximity to the VNA chip 62. The temperature sensor 64 may be a component temperature sensor that measures a component temperature of the glucose sensing module 51, that is, the VNA chip 62, or may be a skin temperature sensor that measures a skin temperature of the arm portion of the user, that is, a body temperature of the user.

The glucose sensing module 51 is provided with at least one of the component temperature sensor that measures the component temperature of the VNA chip 62 or the skin temperature sensor that measures the body temperature of the user (wearer).

In this case, an electrode portion constituting the skin temperature sensor may be arranged, for example, at a portion of the back surface of the sensor substrate 61 in contact with the skin of the arm portion of the user. In addition, for example, the skin temperature sensor may be arranged at a position away from the VNA chip 62 by a predetermined distance or more so as not to be affected by heat generation of the VNA chip 62.

The flash memory 65 is a non-volatile memory, and records information supplied from the VNA chip 62 and the microcomputer 63.

For example, a VNA calibration value which is a calibration value related to the VNA chip 62, a correction coefficient for calibration (correction) related to a glucose level (blood glucose true value), and the like, are recorded in the flash memory 65.

The VNA calibration value is information for correcting deviation from a true value of the S parameter caused for each glucose sensing module 51, that is, an individual difference. For example, the microcomputer 63 or the VNA chip 62 corrects the S parameter obtained by the measurement on the basis of the VNA calibration value, and sets the corrected S parameter as a final S parameter.

The correction coefficient is a coefficient for correcting deviation in a relationship between the S parameter and the glucose level caused by the measurement target, an individual difference of the glucose sensing module 51, a measurement environment, or the like. For example, the microcomputer 63 corrects the glucose level calculated from the S parameter on the basis of the correction coefficient, and sets the corrected glucose level as a final glucose level.

In addition, for example, the glucose level before correction, that is, raw data of the glucose level, the glucose level after correction, that is corrected data of the glucose level, the S parameter, the body temperature of the user, a device temperature of the glucose sensing module 51, or the like, that is, the component temperature, a glucose level reliability parameter, and the like, may also be appropriately recorded in the flash memory 65.

The glucose level reliability parameter is information indicating reliability of the glucose level obtained by the measurement, and is calculated on the basis of, for example, the component temperature, the body temperature of the user, a contact state of the glucose sensing module 51 (sensor electrode) with the user, a body motion state of the user, and the like.

The glucose level reliability parameter may be calculated by the microcomputer 63 or may be calculated by the main CPU 52. In other words, the microcomputer 63 or the main CPU 52 can function as a control unit that calculates the glucose level reliability parameter.

The main CPU 52 controls operation of the glucose sensing module 51 and controls display of various types of information on the display 23.

For example, on the basis of the glucose level reliability parameter, the main CPU 52 determines whether or not the measured glucose level is accurate, that is, whether or not the measured glucose level can be used for an application, or the like.

In addition, for example, the main CPU 52 acquires various types of information such as RAW data and corrected data of the glucose level, the S parameter, the body temperature of the user, and the device temperature (component temperature) from the flash memory 65 via the microcomputer 63, and causes the display 23 to display the acquired information, and the like, or outputs the acquired information, and the like, to an external apparatus.

### <Explanation of Measurement Processing>

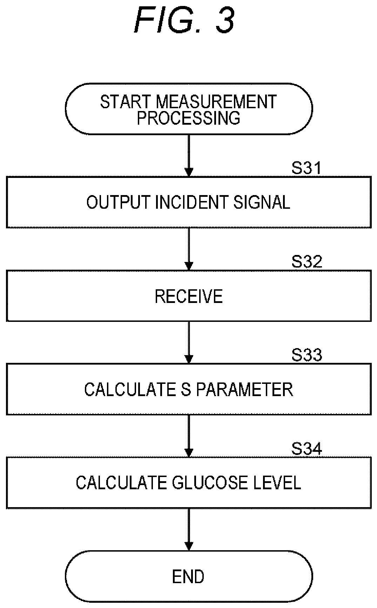

Operation of the smartwatch 11 will be described. In other words, measurement processing by the smartwatch 11 will be described below with reference to the flowchart of Fig. 3.

In step S31, the VNA chip 62 outputs an RF signal of a predetermined frequency, that is, an electromagnetic wave, as the incident signal.

The incident signal is emitted to the body tissue of the arm portion of the user to be measured via the port 1 and the sensor electrode. Then, the incident signal is reflected or transmitted by the body tissue to become a reflected signal or a transmitted signal, and enters the port 1 or the port 2 via the sensor electrode. In addition, the incident signal is received by the VNA chip 62 via a directional coupler provided in the VNA chip 62.

In step S32, the VNA chip 62 receives the reflected signal or the transmitted signal incident from the port 1 or the port 2 via the sensor electrode.

In step S33, the VNA chip 62 calculates the S parameter on the basis of the incident signal and at least the reflected signal between the reflected signal and the transmitted signal, and supplies the S parameter to the microcomputer 63.

In this event, for example, the microcomputer 63 or the VNA chip 62 may perform correction processing on the calculated S parameter based on the VNA calibration value, or the like, recorded in advance in the flash memory 65.

In step S34, the microcomputer 63 calculates a glucose concentration in the blood as a glucose level on the basis of the S parameter supplied from the VNA chip 62.

For example, in step S34, a complex dielectric constant of the measurement target is calculated by a probe method or an S parameter method on the basis of the S parameter, and the glucose level is calculated on the basis of the complex dielectric constant.

In this event, for example, the microcomputer 63 may perform correction processing on the glucose level (RAW data) on the basis of the correction coefficient recorded in the flash memory 65, and use the corrected glucose level (corrected data) as the final glucose level.

The microcomputer 63 supplies the obtained RAW data or corrected data of the glucose level to the flash memory 65 to record the data, or supplies the data to the main CPU 52. If the glucose level is calculated, the measurement processing ends.

As described above, the smartwatch 11 irradiates the measurement target with the incident signal, and calculates the glucose level on the basis of the received reflected signal and transmitted signal.

### <Element Arrangement in Sensor Substrate>

Meanwhile, the glucose sensing module 51 is arranged such that a distance between the VNA chip 62 and the sensor electrode is equal to or less than a predetermined distance in plan view in order to improve measurement accuracy of the S parameter, that is, the measurement accuracy of the glucose level, and to reduce the size. Specifically, the predetermined distance here is 30 mm.

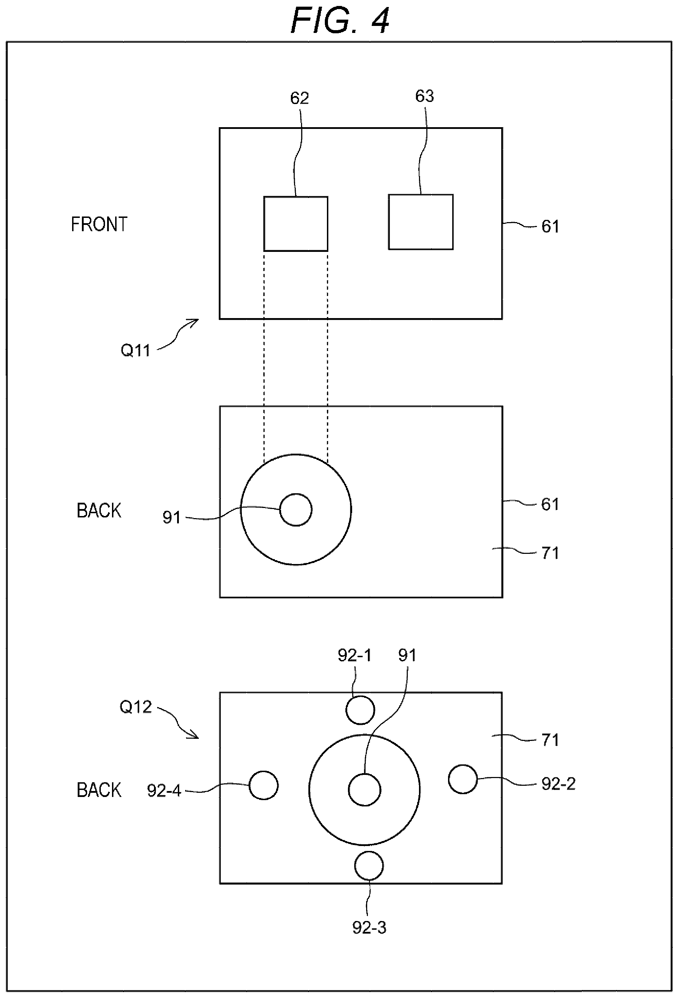

Specifically, for example, as indicated by an arrow Q11 in Fig. 4, the VNA chip 62 and the microcomputer 63 are arranged on the surface (upper surface) of the sensor substrate 61.

Further, the back surface (lower surface) of the sensor substrate 61 is the sensor electrode area 71. In the sensor electrode area 71, a sensor electrode 91 that guides (irradiates) the incident signal output (emitted) from the VNA chip 62 to the arm portion of the user to be measured is arranged.

The sensor electrode 91 functions as a probe electrically connected to the VNA chip 62 and abuts on the arm portion of the user to be measured when the smartwatch 11 is worn.

At the time of measurement, an incident signal output from the VNA chip 62 is emitted from the sensor electrode 91, and the measurement target is irradiated with the incident signal. In addition, a reflected signal corresponding to the irradiation of the incident signal is incident on the sensor electrode 91 and is guided to the VNA chip 62. Note that, in a case where the transmitted signal is also used for measuring the S parameter, the smartwatch 11 is also provided with another sensor electrode 91 that guides the transmitted signal to the VNA chip 62.

In this example, the VNA chip 62 and the sensor electrode 91 are arranged such that the VNA chip 62 overlaps with the sensor electrode 91 when the sensor substrate 61 is viewed from a direction perpendicular to the front surface or the back surface of the sensor substrate 61, that is, when the sensor substrate 61 is viewed in plan view. In other words, the sensor electrode 91 is arranged directly behind the VNA chip 62 in the sensor substrate 61.

In other words, the VNA chip 62 and the sensor electrode 91 are arranged such that a distance between the VNA chip 62 and the sensor electrode 91 is equal to or less than a predetermined distance in plan view.

With such arrangement, a transmission path of the RF signal propagating between the VNA chip 62 and the sensor electrode 91, such as the incident signal and the reflected signal, can be made as short as possible. As a result, loss, that is, attenuation (loss) due to the propagation of the RF signal, or the like, can be reduced as much as possible, and the measurement accuracy of the S parameter (glucose level) can be improved.

Furthermore, for example, as indicated by an arrow Q12, electrode portions 92-1 to 92-4 of a predetermined sensor such as a touch sensor or an impedance sensor may be arranged on the back surface (lower surface) of the sensor substrate 61, that is, in the vicinity of the sensor electrode 91 in the sensor electrode area 71. Note that, hereinafter, the electrode portion 92-1 to the electrode portion 92-4 will be also simply referred to as the electrode portion 92 in a case where it is not particularly necessary to distinguish them.

By arranging the electrode portion 92 in the vicinity of the sensor electrode 91, more accurate sensing can be performed on the arm portion to be measured by the electrode portion 92. By using such a sensing result in the electrode portion 92, more accurate S parameter and glucose level can be obtained, and measurement accuracy can be improved.

The electrode portion 92 may be integrally provided with other portions of the sensor. In other words, the entire sensor including the electrode portion 92 may be arranged in the vicinity of the sensor electrode 91.

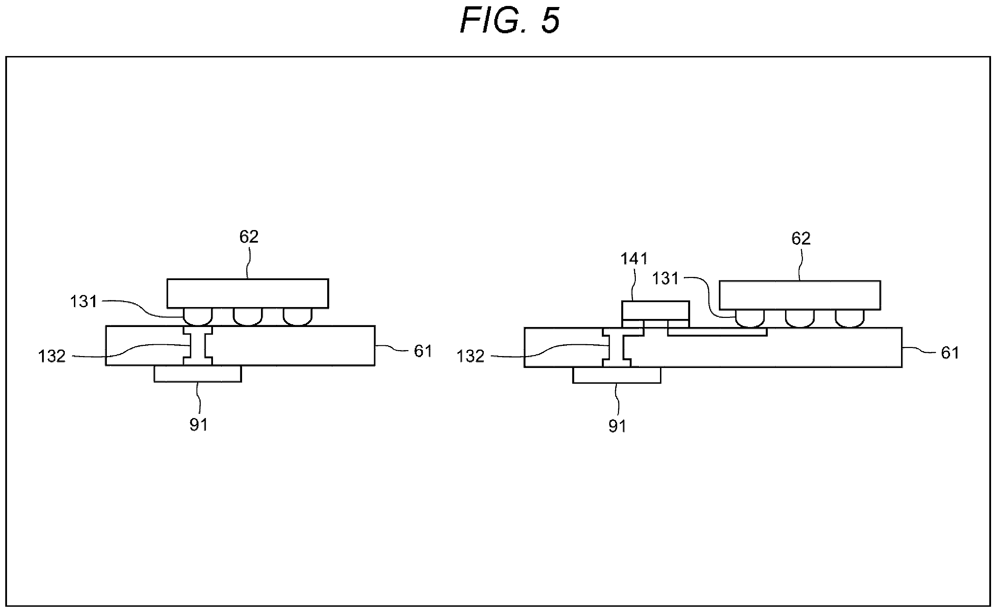

Further, in a case of the arrangement indicated by an arrow Q11, a cross section of the sensor substrate 61 is as illustrated on the left side of Fig. 5, for example. Note that, in Fig. 5, portions corresponding to those in Fig. 4 are denoted by the same reference numerals, and the description thereof will be omitted.

In the example illustrated on the left side of Fig. 5, the VNA chip 62 is mounted on the surface (upper surface) of the sensor substrate 61. In particular, in this example, an RF signal terminal 131 provided on the VNA chip 62 is connected to an in-substrate wiring 132 formed inside the sensor substrate 61.

In addition, in the back surface (lower surface) portion of the sensor substrate 61, the sensor electrode 91 arranged directly behind the VNA chip 62 is connected to the in-substrate wiring 132 of the sensor substrate 61.

In particular, in the sensor substrate 61, the VNA chip 62 (RF signal terminal 131) and the sensor electrode 91 are connected at the shortest distance by the in-substrate wiring 132, that is, linearly connected, so that the in-substrate wiring 132 can be made a linear (or substantially linear) wiring penetrating the sensor substrate 61. For example, a through VIA, or the like, is conceivable as an example of the linear wiring. Note that a shape of the in-substrate wiring 132 is not limited to a linear shape, and may be any other shape.

The RF signal terminal 131 is a terminal that outputs the incident signal (RF signal) and functions as a port of the VNA chip 62.

For example, the incident signal (RF signal) output from the RF signal terminal 131 reaches the sensor electrode 91 through the in-substrate wiring 132. Then, the incident signal is emitted from the sensor electrode 91 to the measurement target that is in contact with the sensor electrode 91.

Further, for example, the RF signal such as the reflected signal incident on the sensor electrode 91 from the measurement target is propagated to the RF signal terminal 131 through the in-substrate wiring 132.

In this example, the RF signal terminal 131 is directly connected to the in-substrate wiring 132. In other words, the RF signal terminal 131 is directly connected to the sensor substrate 61. It is therefore possible to reduce signal loss of the incident signal output from the VNA chip 62 (RF signal terminal 131) and the reflected signal incident on the VNA chip 62 (RF signal terminal 131) from the sensor electrode 91 through the in-substrate wiring 132. As a result, the measurement accuracy of the S parameter (glucose level) can be further improved. Further, by directly connecting the RF signal terminal 131 to the in-substrate wiring 132, a size of the glucose sensing module 51 can be reduced.

Note that in mounting the VNA chip 62, the RF signal terminal 131 and the in-substrate wiring 132 may be connected via a solder ball, that is, connected by a solder ball. Even in such a case, the size of the glucose sensing module 51 can be reduced.

Furthermore, the sensor electrode 91 does not necessarily need to be arranged directly behind the VNA chip 62, and may be arranged such that the distance between the sensor electrode 91 and the VNA chip 62 is equal to or less than the predetermined distance in plan view, for example, arranged at a position in the vicinity of directly behind the VNA chip 62 on the back surface of the sensor substrate 61.

Thus, for example, as illustrated on the right side in the figure, the distance between the sensor electrode 91 and the VNA chip 62 may be 30 mm or less in plan view, and the VNA chip 62 and the sensor electrode 91 may be connected via a predetermined device 141.

For example, a connector with an RF switch, an RF switch, a chip capacitor, a chip resistor, a chip inductor, other passive components, or the like, can be used as the device 141.

In this example, the VNA chip 62 and the device 141 are mounted on the surface (upper surface) of the sensor substrate 61, and the RF signal terminal 131 of the VNA chip 62 and the device 141 are electrically connected by a wiring, or the like, formed on the surface, or the like, of the sensor substrate 61. Note that the RF signal terminal 131 of the VNA chip 62 may be directly connected to the wiring between the sensor substrate 61 and the device 141 (wiring on the sensor substrate 61 or in the sensor substrate 61), or may be connected to the wiring of the sensor substrate 61 via a solder ball.

Further, the device 141 is connected to an end on a side of the upper surface of the in-substrate wiring 132 formed inside the sensor substrate 61, and an end on a side of the lower surface of the in-substrate wiring 132 is connected to the sensor electrode 91. Thus, the device 141 is electrically connected between the VNA chip 62 and the sensor electrode 91.

Also in this example, a propagation path of the RF signal such as the incident signal is made as short as possible, and the measurement accuracy of the S parameter (glucose level) is improved.

In particular, in this example, the device 141 and the sensor electrode 91 are arranged such that the device 141 overlaps with the sensor electrode 91 in plan view. In addition, the device 141 and the sensor electrode 91 are connected by the linear in-substrate wiring 132 penetrating the sensor substrate 61. Thus, the device 141 and the sensor electrode 91 can be connected with the shortest distance. Furthermore, by providing (connecting) an arbitrary device 141 between the VNA chip 62 and the sensor electrode 91, an arbitrary function such as a switch can be added between the VNA chip 62 and the sensor electrode 91.

In the example illustrated on the right side in the figure, the VNA chip 62 outputs the incident signal to the sensor electrode 91 via the device 141 and the in-substrate wiring 132. Similarly, the reflected signal from the measurement target is incident on the VNA chip 62 via the sensor electrode 91, the in-substrate wiring 132, and the device 141.

As described above, in the example illustrated on the right side of Fig. 5, the device 141 overlaps with the sensor electrode 91 in plan view, and the distance between the VNA chip 62 and the sensor electrode 91 is set to be equal to or less than the predetermined distance in plan view, so that the measurement accuracy is improved.

### <Second Embodiment>

### <Determination of Normality of Glucose Level>

In order to accurately measure the glucose level, it is important to perform measurement in a state where the sensor electrode 91 is sufficiently in contact with the arm portion that is the measurement target of the user, that is, in a state where the sensor electrode 91 abuts on the arm portion.

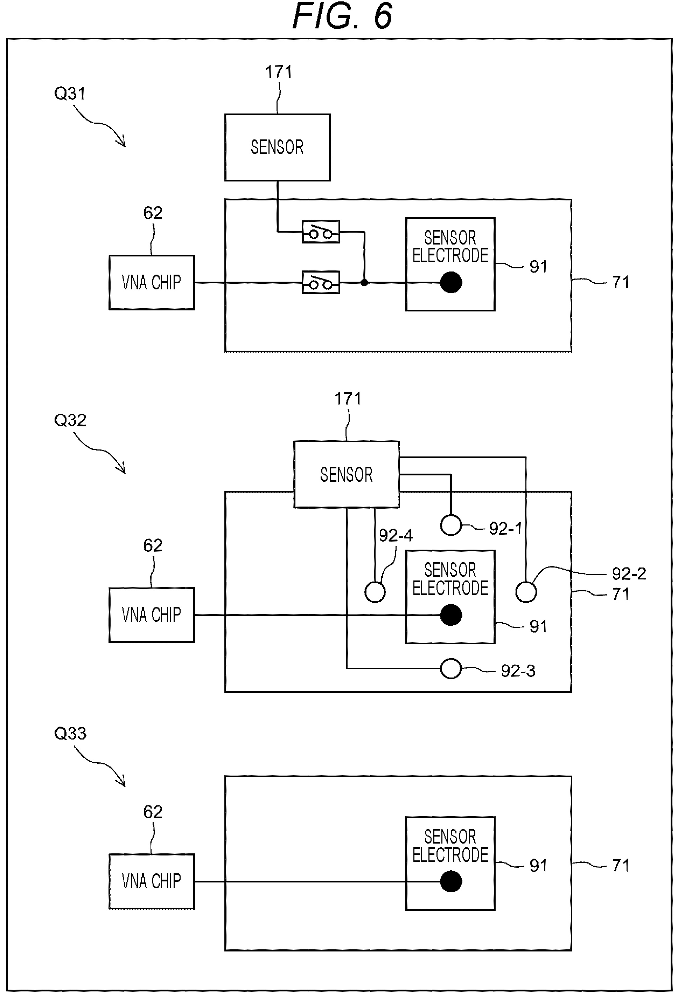

Thus, for example, as illustrated in Fig. 6, whether or not the sensor electrode 91 is in contact with the arm portion may be determined, in other words, normality of the glucose level obtained by the measurement may be determined. Note that, in Fig. 6, portions corresponding to the case in Fig. 4 are denoted by the same reference numerals, and description thereof will be omitted as appropriate.

In the example indicated by an arrow Q31 in Fig. 6, the sensor 171 is provided on the surface (upper surface), or the like, of the sensor substrate 61, and the sensor 171 determines (detects) a contact state of the sensor electrode 91 with respect to the arm portion of the user. In particular, in this example, the sensor 171 is connected to the sensor electrode 91, and sensing is performed on the sensor electrode 91.

Specifically, the sensor 171 includes, for example, a touch sensor or an impedance sensor, and functions as a contact detection sensor that detects a contact state of the sensor electrode 91 with the measurement target by detecting (sensing) a capacitance value or a resistance value of the sensor electrode 91.

In this case, the microcomputer 63 or the main CPU 52 determines whether or not the sensor electrode 91 is in contact with the measurement target, that is, whether or not the glucose level obtained by the measurement is correct, on the basis of the capacitance value and the resistance value obtained as the sensing result by the sensor 171. Then, the microcomputer 63 or the main CPU 52 appropriately records the glucose level in the flash memory 65 or uses the glucose level for a predetermined application, or the like, according to the determination result.

Note that the above-described glucose level reliability parameter may be calculated by the microcomputer 63 or the main CPU 52 on the basis of the capacitance value or the resistance value obtained as the sensing result by the sensor 171. In such a case, whether or not the glucose level is correct is determined on the basis of the calculated glucose level reliability parameter.

In the example indicated by an arrow Q32, the electrode portion 92-1 to the electrode portion 92-4 of the sensor 171 are arranged in the vicinity of the sensor electrode 91 on the back surface (lower surface) of the sensor substrate 61. In other words, the main body portion of the sensor 171 arranged on the front surface, or the like, of the sensor substrate 61 is connected to the electrode portion 92 of the sensor 171 arranged on the back surface of the sensor substrate 61.

Thus, in this example, the sensor 171 detects (senses) the capacitance value and the resistance value in a portion of the electrode portion 92 located in the vicinity of the sensor electrode 91, that is, a portion of the sensor electrode area 71 which abuts on the measurement target.

Then, on the basis of the sensing result, as in the example indicated by the arrow Q31, it is determined whether or not the glucose level is correct, or the glucose level reliability parameter is calculated.

Note that, in the example indicated by the arrow Q31 or the arrow Q32, instead of the normality of the glucose level being determined after the glucose level is measured, whether or not to measure the glucose level (S parameter) may be determined on the basis of the sensing result by the sensor 171.

In this case, for example, the microcomputer 63 or the main CPU 52 functioning as a control unit determines whether or not the sensor electrode 91 is in contact with the measurement target on the basis of the capacitance value or the resistance value obtained as the sensing result by the sensor 171. Then, the glucose level is measured according to the determination result.

In the example indicated by the arrow Q33, the sensor substrate 61 is not provided with a sensor for determining the normality of the glucose level, and normality determination, that is, whether the sensor electrode 91 is in contact with the measurement target is performed on the basis of S11 calculated as the S parameter by the VNA chip 62.

In this case, for example, the microcomputer 63 or the main CPU 52 functioning as a control unit calculates impedance of the sensor electrode 91 on the basis of S11 which is the S parameter obtained by the VNA chip 62.

Then, the microcomputer 63 or the main CPU 52 determines whether or not the sensor electrode 91 is in contact with the measurement target, that is, the contact state of the sensor electrode 91 with the measurement target on the basis of the obtained impedance. In other words, it is determined whether or not the glucose level obtained by the measurement is correct.

As described above, by determining the normality of the glucose level, it is possible to use only the measurement result obtained in a state where the measurement target is correctly in contact with the sensor electrode 91. In other words, the measurement accuracy of the glucose level (S parameter) can be improved.

### <Third Embodiment>

### <Use of Body Temperature Detection>

The smartwatch 11 can provide a biological sensing solution for sensing the biological information of the user such as the glucose level (blood glucose level) by acquiring (calculating) the S parameter by the VNA chip 62.

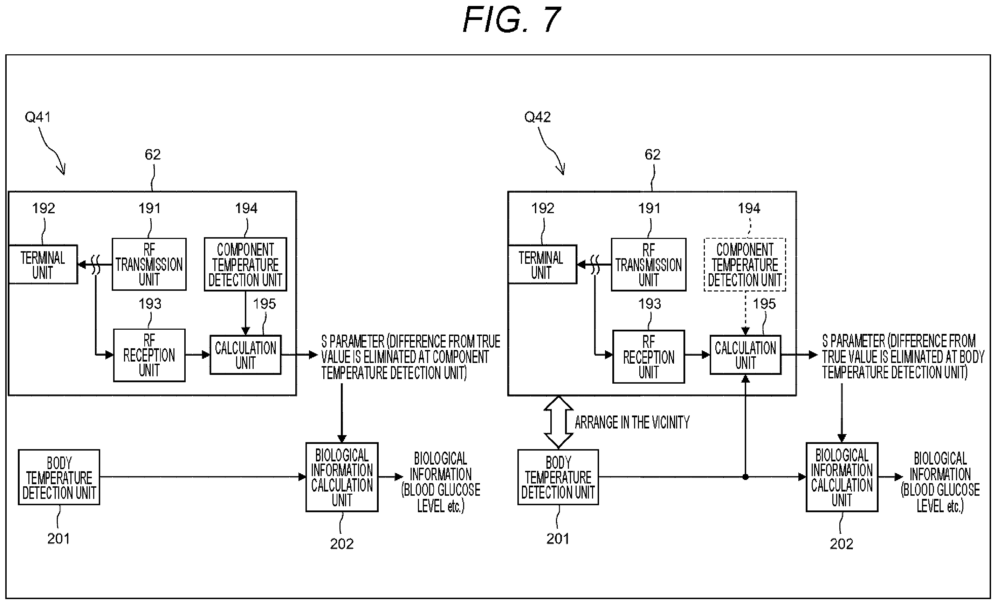

It is conceivable that the VNA chip 62 is provided with, for example, an RF transmission unit 191, a terminal unit 192, an RF reception unit 193, a component temperature detection unit 194, and a calculation unit 195 as indicated by an arrow Q41 in Fig. 7.

In this example, the RF transmission unit 191 generates an RF signal of a predetermined frequency as the incident signal, and transmits (outputs) the incident signal to the sensor electrode 91 connected to the terminal unit 192 via a directional coupler (not illustrated) or the terminal unit 192. The terminal unit 192 functions as a port and corresponds to, for example, the RF signal terminal 131 illustrated in Fig. 5.

The incident signal output from the terminal unit 192 is emitted to the arm portion of the user as the measurement target via the sensor electrode 91.

The incident signal radiated to the measurement target becomes a reflected signal by reflection, and the reflected signal is incident on the RF reception unit 193 via the sensor electrode 91, the terminal unit 192, and the directional coupler (not illustrated). The RF reception unit 193 receives the reflected signal incident from the directional coupler. In other words, the VNA chip 62 receives the reflected signal of the RF signal transmitted (output) by itself as the incident signal.

In addition, part of the incident signal output from the RF transmission unit 191 is also incident on the RF reception unit 193 via the directional coupler (not illustrated) and is received by the RF reception unit 193.

The RF reception unit 193 supplies the received incident signal, more specifically, a digital signal obtained by performing analog to digital (AD) conversion on an analog signal obtained by receiving the incident signal, to a calculation unit 195. Similarly, the RF reception unit 193 supplies a digital signal obtained by performing AD conversion on the received reflected signal, more specifically, an analog signal obtained by receiving the reflected signal, to the calculation unit 195. Note that the port 2 is also used, and in a case where the transmitted signal is received, the transmitted signal is also supplied to the calculation unit 195.

The component temperature detection unit 194 includes a component temperature sensor, detects the temperature (component temperature) of the VNA chip 62, and supplies the detection result to the calculation unit 195.

The calculation unit 195 calculates the S parameter on the basis of the incident signal and the reflected signal supplied from the RF reception unit 193.

In this event, the calculation unit 195 performs correction processing on the S parameter on the basis of the component temperature supplied from the component temperature detection unit 194 and the VNA calibration value recorded in the flash memory 65. Then, the corrected S parameter is output as the final S parameter.

By such correction (calibration), it is possible to eliminate occurrence of an error from a true value of the S parameter due to the temperature, the individual difference, or the like, of the VNA chip 62 and to obtain a more accurate S parameter.

Further, the sensor substrate 61 is also provided with a body temperature detection unit 201. The body temperature detection unit 201 includes a temperature sensor, and corresponds to, for example, a temperature sensor 64 illustrated in Fig. 2.

The body temperature detection unit 201 detects (senses) the skin temperature of the arm portion of the user to be measured, that is, the body temperature of the user, and supplies the detection result to the biological information calculation unit 202.

The biological information calculation unit 202 is implemented by, for example, the microcomputer 63. The biological information calculation unit 202 calculates the biological information such as the glucose level on the basis of the skin temperature (body temperature) supplied from the body temperature detection unit 201 and the S parameter supplied from the calculation unit 195, and outputs the biological information to the subsequent stage.

In the example indicated by an arrow Q41, the body temperature detection unit 201 that detects the temperature (temperature of the human body) of the arm portion to be measured by the user is provided in order to calculate the biological information.

Furthermore, if there is a difference between the temperature (hereinafter, also referred to as a measured temperature) of the VNA chip 62 at the time of actual measurement of the S parameter, that is, at the time of acquisition (reception) of the incident signal and the reflected signal, and the temperature (hereinafter, also referred to as a calibration temperature) of the VNA chip 62 at the time of measurement (determination) of the VNA calibration value to be used for correction (calibration) of the S parameter, a difference from the true value occurs in the calculated S parameter.

Thus, in the example indicated by the arrow Q41, by providing the component temperature detection unit 194 in the VNA chip 62, it is possible to acquire the actual measurement temperature, and occurrence of a difference from the true value as described above is suppressed at the time of calculation in the calculation unit 195. In other words, the VNA calibration value is appropriately corrected on the basis of the actual measured temperature, and the S parameter is corrected using the corrected VNA calibration value.

However, if the component temperature detection unit 194 is provided in the VNA chip 62, mounting cost increases accordingly, or a mounting space is required.

Thus, in the present technology, for example, as indicated by an arrow Q42, the body temperature detection unit 201 is arranged in the vicinity of the VNA chip 62, and the detection result of the body temperature (skin temperature) by the body temperature detection unit 201 is used for correction (calibration) of the S parameter.

This eliminates the necessity to provide the component temperature detection unit 194, so that it is possible to reduce the mounting cost and achieve space saving. In addition, a more accurate S parameter having a small difference from the true value can be obtained. In other words, it is possible to improve the measurement accuracy of the biological information (S parameter) while reducing the mounting cost and achieving space saving.

In the example indicated by the arrow Q42, the VNA chip 62 is provided with the RF transmission unit 191, the terminal unit 192, the RF reception unit 193, and the calculation unit 195, and is not provided with the component temperature detection unit 194.

Furthermore, the body temperature detection unit 201 is provided in the vicinity of the VNA chip 62, that is, at a position equal to or less than a predetermined distance from the VNA chip 62. Thus, the detection result obtained by the body temperature detection unit 201 can be used as both the skin temperature (body temperature) to be measured and the component temperature of the VNA chip 62.

Thus, in this example, the temperature obtained by the body temperature detection unit 201 is supplied to the calculation unit 195 and the biological information calculation unit 202.

The calculation unit 195 calculates the S parameter on the basis of the incident signal, the reflected signal, and the skin temperature (body temperature).

More specifically, the calculation unit 195 calculates the S parameter on the basis of the incident signal and the reflected signal supplied from the RF reception unit 193.

In this event, the calculation unit 195 performs correction processing on the S parameter on the basis of the skin temperature (body temperature) supplied from the body temperature detection unit 201 and the VNA calibration value recorded in the flash memory 65. In other words, the VNA calibration value is appropriately corrected on the basis of the skin temperature, and the correction processing for the S parameter is performed using the corrected VNA calibration value. Then, the corrected S parameter is output as the final S parameter.

By such correction (calibration), in a similar manner to the example indicated by the arrow Q41, occurrence of an error from the true value of the S parameter due to the temperature of the VNA chip 62, or the like, can be eliminated, and a more accurate S parameter can be obtained. In other words, the measurement accuracy of the biological information (S parameter) can be improved.

In addition, the biological information calculation unit 202 calculates the biological information such as the glucose level on the basis of the skin temperature (body temperature) supplied from the body temperature detection unit 201 and the S parameter supplied from the calculation unit 195. Note that the biological information calculation unit 202 may appropriately correct the biological information on the basis of the correction coefficient recorded in the flash memory 65, for example. In this event, the skin temperature (body temperature) supplied from the body temperature detection unit 201 may also be used to correct the biological information.

The skin temperature obtained by the body temperature detection unit 201 and the temperature (component temperature) of the VNA chip 62 obtained by the temperature sensor 64 illustrated in Fig. 2 may be used for calculation of the glucose level reliability parameter by the microcomputer 63 or the main CPU 52 functioning as a control unit.

### <Fourth Embodiment>

### <Heat Dissipation of VNA Chip>

As described above, if a difference occurs between the measured temperature and the calibrated temperature, a difference occurs between the calculated S parameter and the true value. In particular, the temperature of the VNA chip 62 at the time of actual measurement may rise due to self-heating, and such temperature rise is one of factors that causes a difference (error) from the true value.

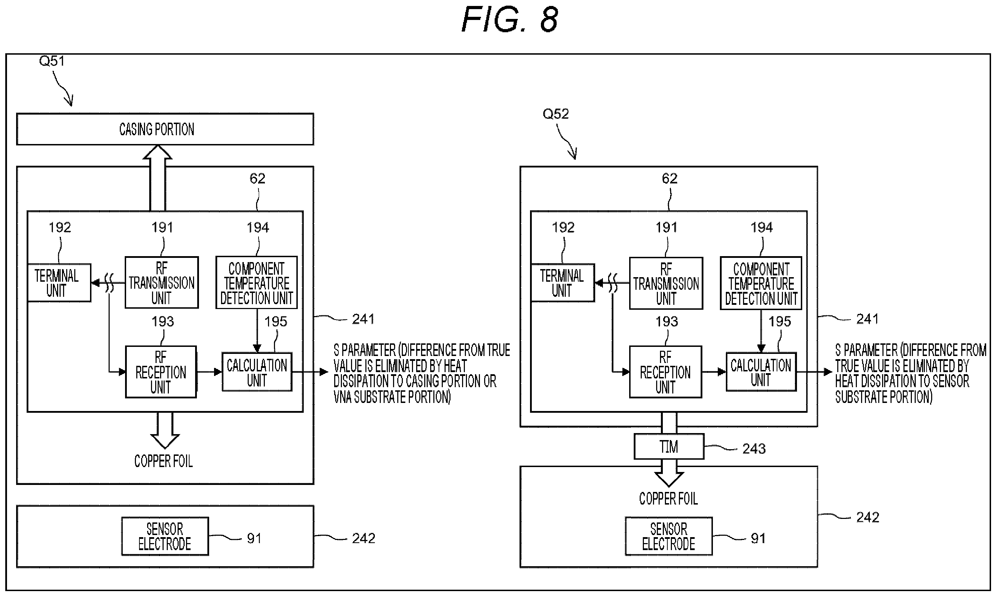

Thus, for example, as indicated by an arrow Q51 in Fig. 8, it is conceivable to diffuse (dissipate) heat generated in the VNA chip 62 to a VNA substrate portion 241 constituting the sensor substrate 61 or a casing portion of the smartwatch 11. Note that, in Fig. 8, portions corresponding to those in the case of Fig. 7 are denoted by the same reference signs, and the description thereof will be omitted as appropriate.

In the example indicated by the arrow Q51, the sensor substrate 61 does not include one semiconductor substrate as in the example illustrated in Fig. 5, but the sensor substrate 61 includes the VNA substrate portion 241 and a sensor substrate portion 242.

In other words, the VNA substrate portion 241 and the sensor substrate portion 242 each includes a semiconductor substrate, and one sensor substrate 61 is formed by joining the VNA substrate portion 241 and the sensor substrate portion 242.

In particular, in this example, the VNA chip 62 is arranged (mounted) on the VNA substrate portion 241, and the sensor electrode 91 is provided on the sensor substrate portion 242.

In this example, it is possible to suppress the temperature rise of the VNA chip 62 by diffusing the heat generated by the self-heating in the VNA chip 62 to the casing portion of the smartwatch 11 and the VNA substrate portion 241. However, in such a case, it is necessary to consider specifications of the casing portion and specifications of the back surface side of the VNA chip 62.

Thus, in the present technology, the temperature rise of the VNA chip 62 can be more easily suppressed by arranging a thermal interface material (TIM) 243 between the surface of the VNA chip 62 and a copper foil provided on the surface (upper surface) of the sensor substrate portion 242 as indicated by an arrow Q52, for example.

In this example, the surface (upper surface) of the VNA chip 62 mounted on the VNA substrate portion 241 is in contact with the surface (upper surface) of the sensor substrate portion 242 via the TIM 243.

In addition, the copper foil is provided on a surface (surface layer) of the sensor substrate portion 242 on a side of the VNA substrate portion 241, that is, a contact surface of the sensor substrate portion 242 with the VNA substrate portion 241 via the TIM 243.

In this example, the VNA chip 62 is in contact with the copper foil on the surface of the sensor substrate portion 242 via the TIM 243 which is a thermally conductive material.

As a result, efficiency of thermal diffusion (heat transfer efficiency) can be improved, and the temperature rise of the VNA chip 62 can be suppressed. In particular, in this example, it is only necessary to provide the TIM 243, and thus, it is not necessary to consider the specifications of the casing portion and the specifications of the back surface side of the VNA chip 62, and the temperature rise of the VNA chip 62 can be more easily suppressed.

Furthermore, by suppressing the temperature rise of the VNA chip 62, it is possible to eliminate occurrence of an error from the true value of the S parameter due to the temperature of the VNA chip 62 and to obtain a more accurate S parameter. In other words, the measurement accuracy of the biological information (S parameter) can be improved.

### <Fifth Embodiment>

### <Correction of S Parameter>

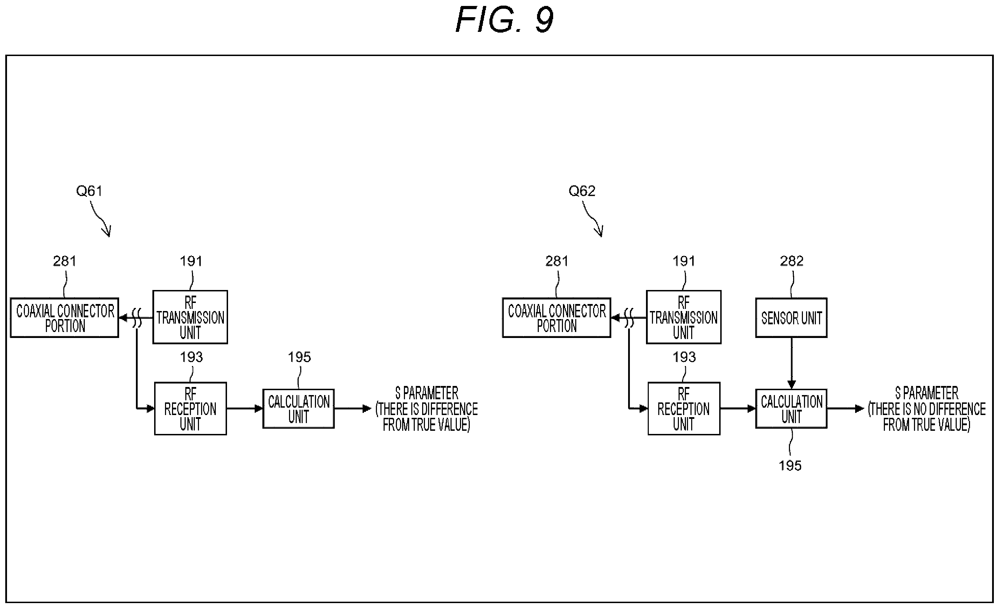

For transmission and reception of the RF signal that becomes then incident signal or the reflected signal, that is, transmission of the incident signal from the RF transmission unit 191 of the VNA chip 62 to the sensor electrode 91 and transmission of the reflected signal from the sensor electrode 91 to the RF reception unit 193, for example, as illustrated in Fig. 9, it is conceivable to use a coaxial connector portion 281. Note that, in Fig. 9, portions corresponding to the case in Fig. 7 are denoted by the same reference numerals, and description thereof will be omitted as appropriate.

For example, in an example indicated by an arrow Q61 in Fig. 9, each of the RF transmission unit 191 and the RF reception unit 193 is connected to a directional coupler (not illustrated), and the directional coupler is connected to the coaxial connector portion 281 having a coaxial connector. Further, the sensor electrode 91 is connected to the coaxial connector portion 281 on a side opposite to a side of the directional coupler.

More specifically, for example, the coaxial connector portion 281 is arranged between the in-substrate wiring 132 of the sensor substrate 61 and the sensor electrode 91 in the example illustrated in Fig. 5.

In the example indicated by the arrow Q61, the incident signal output from the RF transmission unit 191 is emitted to the arm portion of the user to be measured via the directional coupler, the coaxial connector portion 281, and the sensor electrode 91.

In addition, the reflected signal generated by irradiation of the incident signal is incident on the RF reception unit 193 via the sensor electrode 91, the coaxial connector portion 281, and the directional coupler.

Furthermore, the calculation unit 195 calculates the S parameter on the basis of the incident signal and the reflected signal supplied from the RF reception unit 193.

Meanwhile, vibration may be applied to a contact inside the coaxial connector portion 281. For example, in the smartwatch 11 that measures biological information, vibration is applied to the coaxial connector portion 281 (contact) by body motion such as walking or running of the user who is the wearer of the smartwatch 11.

In the configuration indicated by the arrow Q61, if measurement of the biological information such as the glucose level, that is, measurement of the S parameter is performed in a state where vibration is applied to the coaxial connector portion 281 (contact) due to the body motion of the user, or the like, the transmission path, the transmission characteristics, and the like, of the RF signal change. Then, a difference from the true value occurs in the S parameter obtained at the time of measurement.

Thus, in the present technology, for example, as indicated by an arrow Q62, the sensor unit 282 that detects body motion such as a walking state or a running state of the user is provided, and the S parameter is calculated using the sensing result of the sensor unit 282. By doing so, it is possible to suppress influence of the body motion of the user to be low and to obtain an accurate S parameter closer to the true value.

In the example indicated by the arrow Q62, in a similar manner to the example indicated by the arrow Q61, the coaxial connector portion 281 is provided between the directional coupler to which the RF transmission unit 191 and the RF reception unit 193 are connected and the sensor electrode 91. The coaxial connector portion 281 is provided between the sensor electrode 91 and the VNA chip 62 in the sensor substrate 61.

Furthermore, in the example indicated by the arrow Q62, the sensor unit 282 is provided as a body motion detection unit that detects a body motion state of the user who is the wearer of the smartwatch 11.

The sensor unit 282 is a body motion sensor, and is provided, for example, on the surface of the sensor substrate 61 or in a portion other than the sensor substrate 61 inside the main body 21.

The sensor unit 282 detects (senses) the body motion of the user, and supplies information (hereinafter, also referred to as body motion information) indicating the body motion state of the user obtained as a result to the calculation unit 195.

The calculation unit 195 calculates the S parameter on the basis of the incident signal, the reflected signal, and the body motion information.

More specifically, the calculation unit 195 calculates the S parameter on the basis of the incident signal and the reflected signal supplied from the RF reception unit 193. In this event, the calculation unit 195 performs correction processing on the S parameter on the basis of the body motion information supplied from the sensor unit 282, and outputs the corrected S parameter to the biological information calculation unit 202 as the final S parameter.

Note that, in the calculation unit 195, as in the example described with reference to Fig. 7, and the like, S parameter correction processing may be performed using the skin temperature, the component temperature, and the VNA calibration value.

With such correction based on the body motion information, it is possible to eliminate occurrence of an error from the true value of the S parameter caused by the vibration applied to the coaxial connector portion 281 by the body motion of the user and to obtain a more accurate S parameter. In other words, the measurement accuracy of the biological information (S parameter) can be improved.

Note that the body motion information obtained by the sensor unit 282 may be used for calculation of the glucose level reliability parameter by the microcomputer 63 or the main CPU 52 functioning as the control unit.

### <Sixth embodiment>

### <Setting of Average Number of Times>

By the way, in a VNA chip to be used for a soil moisture sensor or a biological information sensor, a technique of obtaining an average value of S parameters in order to obtain desired measurement accuracy, that is, a desired signal to noise ratio (SNR) has been proposed.

In this technique, the average number of times, which is the number of S parameters to be used to calculate the average value, that is, the number of times of performing a series of processing of calculating the S parameters by emitting an incident signal and receiving a reflected signal is uniformly set regardless of a frequency band of the incident signal.

In order to improve measurement accuracy, it is necessary to increase the average number of times, but increasing the average number of times leads to increase in a measurement period and increase in power consumption.

In addition, accuracy required for measurement differs for each frequency band, and thus, in a case where the average number of times is set uniformly in all the frequency bands, it is necessary to adjust the average number of times at each frequency to the average number of times of a frequency band in which a large average number of times is required. This is disadvantageous in terms of power consumption and a measurement period.

Thus, in the present technology, the average number of times can be set (set) in advance for each frequency of the incident signal, so that measurement accuracy can be improved while suppressing increase in a measurement period and power consumption.

In the VNA module of the present technology, the S parameter of each frequency, that is, each channel corresponding to each frequency is measured with reference to the average number of times set (set) in advance. Hereinafter, the frequency of the incident signal is also referred to as a channel. In other words, the incident signal of a predetermined frequency is also referred to as an incident signal of a predetermined channel.

As a method for setting the average number of times for each frequency (channel) in the present technology (hereinafter, also referred to as a method for setting an average number of times), for example, the following three methods are conceivable.

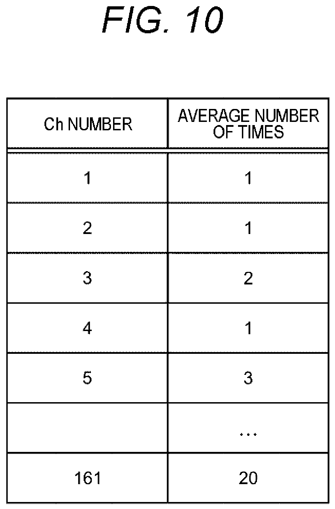

First, a first method for setting an average number of times is a method in which a unique average number of times is set for each channel (frequency) as indicated in Fig. 10.

In Fig. 10, a field of "Ch number" indicates a channel number indicating the channel of the incident signal, and a field of "average number of times" indicates an average number of times in the channel indicated by the channel number.

For example, the average number of times of the channel with the channel number "3" is set to "2". Thus, in this channel, a series of processing including emission (irradiation) of the incident signal, reception of the reflected signal, and calculation of the S parameter is performed twice. Then, an average value of the obtained two S parameters is obtained, and the average value is output as the final S parameter obtained by the measurement.

Hereinafter, as in the example of Fig. 10, a table in which the channel number of each channel and the average number of times in each channel are stored in association with each other will be also referred to as a table of an average number of times for each channel. It can be said that the table of the average number of times for each channel is information indicating the average number of times for each channel of the RF signal (incident signal) with which the measurement target is irradiated.

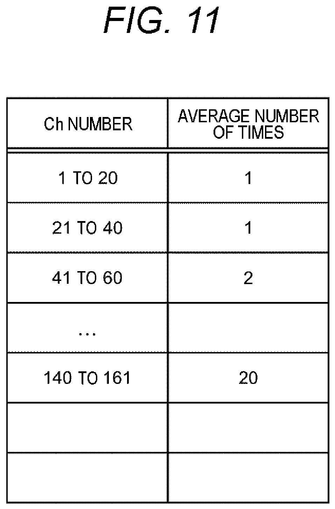

A second method for setting the average number of times is a method in which a channel (frequency) is divided into several regions (bands), and a unique average number of times is set for each region as indicated in Fig. 11.

In the example of Fig. 11, one or a plurality of continuous channels are described in the field of "Ch number", and a group including these channels is set as one channel region. Furthermore, in a field of "average number of times", the average number of times in each channel belonging to the channel region is indicated.

Thus, in this example, the average number of times can be set for each channel region, and the same average number of times is set for all channels belonging to one channel region.

In the second method for setting the average number of times, the average number of times is determined for each one or a plurality of channels, that is, for each frequency band, so that the setting can be performed more easily.

Hereinafter, as in the example of Fig. 11, a table in which the channel number of each channel belonging to the channel region and the average number of times in the channel region are stored in association with each other will be also referred to as a table of an average number of times for each channel region. It can be said that the table of the average number of times for each channel region is information indicating the average number of times for each channel region including one or a plurality of channels of the RF signal (incident signal) with which the measurement target is irradiated.

A third method for setting the average number of times is a setting method combining the first method for setting the average number of times and the second method for setting the average number of times described above.

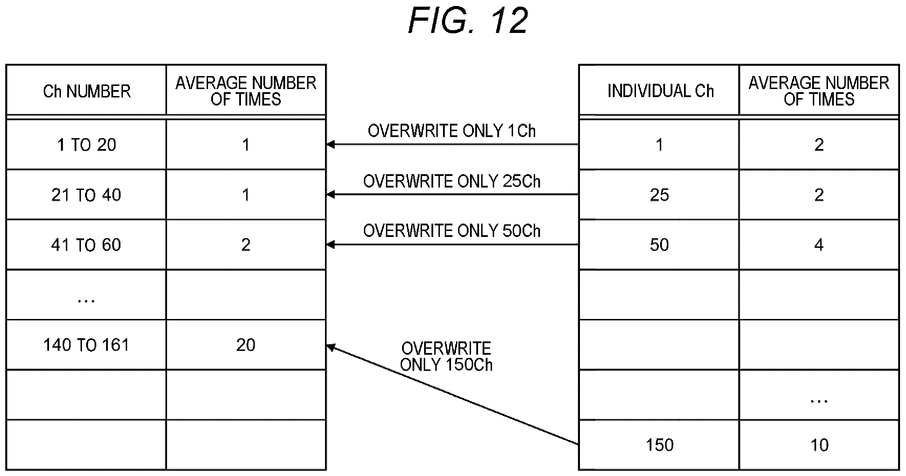

In the third method for setting the average number of times, for example, not only the average number of times can be set for each channel region as indicated in Fig. 12, but also an exceptional channel among the channels belonging to the channel region and the average number of times of the exceptional channel can be set.

In other words, the table of the average number of times for each channel region indicating the average number of times for each channel region is indicated on the left side of Fig. 12.

In addition, a table in which the channel number (individual Ch) of the channel that becomes exceptional (hereinafter, also referred to as an exceptional channel) is associated with the average number of times in the exceptional channel indicated by the channel number is indicated on the right side in the drawing. Hereinafter, a table in which the channel number of each exceptional channel and the average number of times in each exceptional channel are stored in association with each other will be also referred to as a table of an average number of times for each exceptional channel.

In the third method for setting the average number of times, the average number of times is determined with reference to the table of the average number of times for each channel region and the table of the average number of times for each exceptional channel.

In other words, in the third method for setting the average number of times, the average number of times can be set for each channel region by dividing a plurality of continuous channels into several channel regions, but for an exceptional channel, overwriting can be performed the average number of times for each individual channel.

Specifically, focusing on a first channel region in the table of the average number of times for each channel region indicated on the left side in the drawing, the channels belonging to the channel region (hereinafter, also referred to as a channel region of interest) are channels with channel numbers "1" to "20". Further, the average number of times of the channel region of interest is set to "1".

Furthermore, referring to the table of the average number of times for each exceptional channel indicated on the right side in the drawing, among the channels belonging to the channel region of interest, the exceptional channel is the channel with the channel number "1", and the average number of times of the channel is "2".

Thus, at the time of actual measurement, the processing is executed the average number of times "2" for the channel with the channel number "1", and the processing is executed the average number of times "1" for each channel with the channel numbers "2" to "20".

It can be said that the above set of the table of the average number of times for each channel region and the table of the average number of times for exceptional channel is information indicating the average number of times for each channel region including one or a plurality of channels of the RF signal (incident signal) with which the measurement target is irradiated and the average number of times of exceptional channels among the channels belonging to the channel region.

### <Configuration Example of Measurement Apparatus>

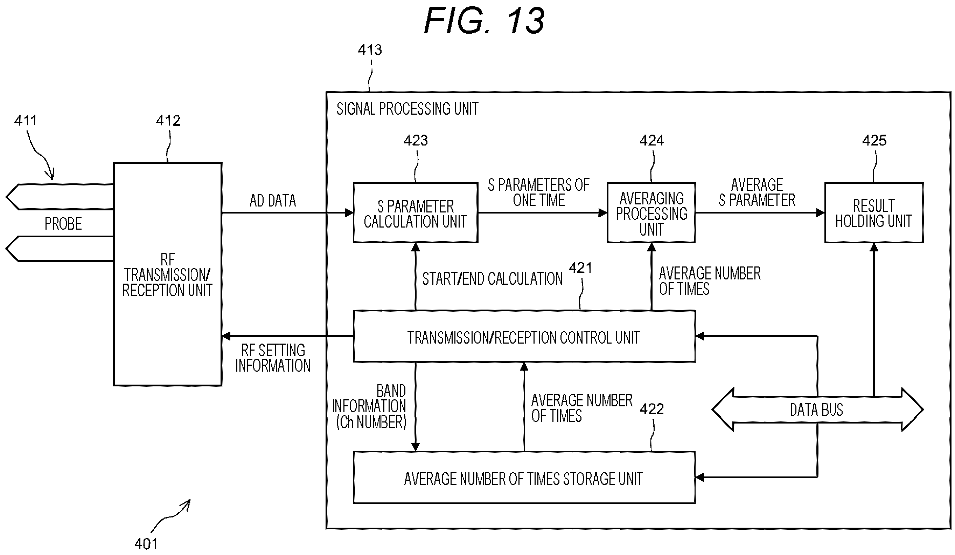

Fig. 13 is a view illustrating a configuration example of an embodiment of a measurement apparatus to which the present technology is applied.

A measurement apparatus 401 illustrated in Fig. 13 includes a VNA module that measures an S parameter, and is mounted on a sensor system that implements, for example, a soil moisture sensor, a biological information sensor, or the like.

The measurement apparatus 401 includes a probe 411, an RF transmission/reception unit 412, and a signal processing unit 413.

In this example, a block including the RF transmission/reception unit 412 and the signal processing unit 413 corresponds to the VNA chip 62 illustrated in Fig. 2, and the RF transmission/reception unit 412 and the signal processing unit 413 are implemented by, for example, one VNA chip (VNA integrated circuit). Note that the RF transmission/reception unit 412 and the signal processing unit 413 may be provided in the VNA chip 62 illustrated in Fig. 2. Furthermore, part of the configuration of the signal processing unit 413 may be provided outside the VNA chip 62.

The probe 411 corresponds to, for example, the sensor electrode 91 illustrated in Fig. 4. At the time of measurement, the probe 411 is arranged so as to abut on (in contact with) a measurement target such as a site such as an arm of the user, irradiates the measurement target with an incident signal incident from the RF transmission/reception unit 412, and guides a reflected signal and a transmitted signal incident from the measurement target to the RF transmission/reception unit 412.

The RF transmission/reception unit 412 corresponds to, for example, a block including components from the RF transmission unit 191 to the RF reception unit 193 illustrated in Fig. 7.

The RF transmission/reception unit 412 outputs an RF signal having a frequency corresponding to the channel as an incident signal for each channel on the basis of the RF setting information supplied from the signal processing unit 413, and irradiates the measurement target with the incident signal via the probe 411. The RF setting information is, for example, information indicating a channel (frequency) to be used for measurement.

Furthermore, the RF transmission/reception unit 412 receives the output incident signal, and receives a reflected signal and a transmitted signal incident from the probe 411 in response to irradiation of the incident signal.

The RF transmission/reception unit 412 performs AD conversion on the received incident signal, reflected signal, and transmitted signal, and supplies digital signals obtained as a result to the signal processing unit 413 as AD data. More specifically, the digital signals obtained by performing AD conversion on an analog signal obtained by receiving the incident signal, the reflected signal, and the transmitted signal are used as AD data.

Hereinafter, the digital signals corresponding to the incident signal, the reflected signal, and the transmitted signal constituting the AD data will be also simply referred to as the incident signal, the reflected signal, and the transmitted signal. In addition, the AD data only needs to include the incident signal and the reflected signal, and does not necessarily need to include the transmitted signal.

The signal processing unit 413 calculates the S parameter on the basis of the AD data supplied from the RF transmission/reception unit 412. The signal processing unit 413 includes a transmission/reception control unit 421, an average number of times storage unit 422, an S parameter calculation unit 423, an averaging processing unit 424, and a result holding unit 425.

Furthermore, the transmission/reception control unit 421, the average number of times storage unit 422, and the result holding unit 425 are connected to each other by a data bus, or the like, and can exchange various types of information.

The transmission/reception control unit 421 controls the entire operation of the signal processing unit 413, such as transmission/reception of the RF signal and calculation of the S parameter.

For example, the transmission/reception control unit 421 generates RF setting information indicating a channel to be measured, supplies the RF setting information to the RF transmission/reception unit 412, and instructs the S parameter calculation unit 423 to start or end the calculation of the S parameter.

In addition, the transmission/reception control unit 421 supplies the channel number of the channel to be measured to the average number of times storage unit 422 as band information, supplies the average number of times supplied from the average number of times storage unit 422 in response to the supply of the band information to the averaging processing unit 424, and gives an instruction to execute the averaging processing for calculating the average value.

The average number of times storage unit 422 stores average number of times information for obtaining the average number of times of each channel (frequency) set in advance. The average number of times information can be updated by control of an upper host, or the like, of the measurement apparatus 401.

For example, the table of the average number of times for each channel, the table of the average number of times for each channel region, the table of the average number of times for each exceptional channel, and the like, are stored as the average number of times information.

Note that the average number of times information is not limited to information in a table format such as the table of the average number of times for each channel, and may be information in any format as long as it is information that can obtain the average number of times in the channel for the channel (frequency) of the incident signal.

Upon receiving supply of the band information from the transmission/reception control unit 421, the average number of times storage unit 422 reads the average number of times corresponding to the channel (channel number) indicated by the band information from the average number of times information and supplies the average number of times to the transmission/reception control unit 421.

Under the control of the transmission/reception control unit 421, the S parameter calculation unit 423 calculates the S parameter on the basis of the AD data supplied from the RF transmission/reception unit 412, and supplies the S parameter to the averaging processing unit 424.

The averaging processing unit 424 calculates an average value of the S parameters of the same channel supplied from the S parameter calculation unit 423 on the basis of the average number of times supplied from the transmission/reception control unit 421, and supplies the calculated average value to the result holding unit 425 as the final S parameter.

In other words, the averaging processing unit 424 calculates, for each channel, the average value of the S parameters as many as the average number of times indicated by the average number of times information.

Hereinafter, the average value of the S parameters will be also referred to as an average S parameter. The average S parameter is the final S parameter obtained for the channel to be processed.

The result holding unit 425 holds (stores) the average S parameter of each channel supplied from the averaging processing unit 424. The average S parameter of each channel held in the result holding unit 425 is supplied to a subsequent block such as the biological information calculation unit 202 illustrated in Fig. 7, or the like, for example, and is used for calculation of the biological information such as the glucose level.

### <Explanation of Measurement Processing>

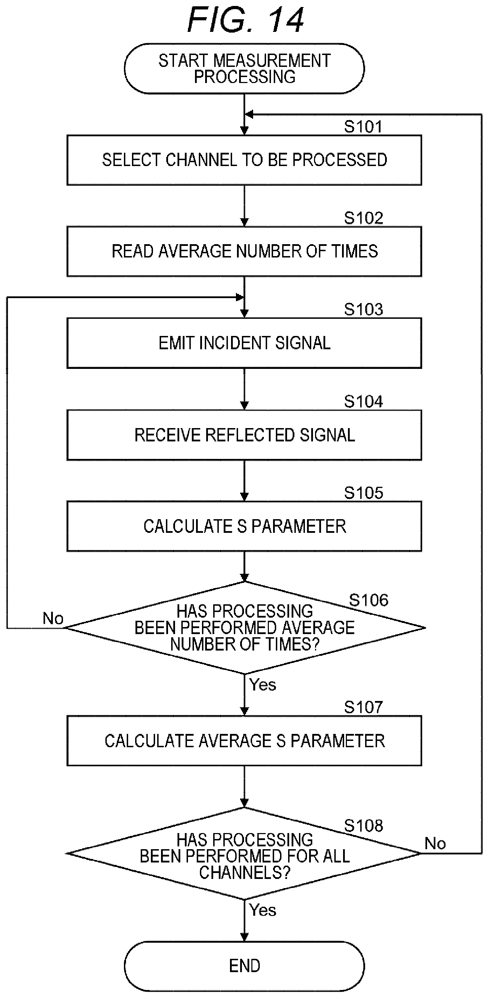

Operation of the measurement apparatus 401 will be described. In other words, measurement processing by the measurement apparatus 401 will be described below with reference to the flowchart of Fig. 14.

In step S101, the transmission/reception control unit 421 selects one channel to be processed from the plurality of channels.

In step S102, the transmission/reception control unit 421 reads the average number of times of the channel selected as the channel to be processed.

For example, the transmission/reception control unit 421 generates band information indicating the channel number of the channel to be processed and supplies the band information to the average number of times storage unit 422, thereby acquiring the average number of times of the channel indicated by the band information from the average number of times storage unit 422.

Specifically, for example, it is assumed that the table of the average number of times for each channel indicated in Fig. 10 is stored in advance as the average number of times information in the average number of times storage unit 422, and the channel number of the channel to be processed indicated by the band information is "3".

In this case, the average number of times storage unit 422 refers to the table of the average number of times for each channel, and supplies "2" to the transmission/reception control unit 421 as the average number of times of the channel to be processed.

Note that, although the first method for setting the average number of times has been described as an example, the average number of times may be read on the basis of the average number of times information set by the second method for setting the average number of times, the third method for setting the average number of times, or other methods.

The transmission/reception control unit 421 generates RF setting information indicating the channel to be processed, and supplies the RF setting information to the RF transmission/reception unit 412.

In addition, the transmission/reception control unit 421 instructs the S parameter calculation unit 423 to start calculation of the S parameter, and supplies the average number of times of the channel to be processed supplied from the average number of times storage unit 422 to the averaging processing unit 424 to give an instruction to execute the averaging processing.

In step S103, the RF transmission/reception unit 412 generates an incident signal of the channel to be processed on the basis of the RF setting information supplied from the transmission/reception control unit 421, and irradiates the measurement target with the incident signal.

Specifically, the RF transmission/reception unit 412 generates an RF signal having a frequency corresponding to the channel indicated by the RF setting information as the incident signal, and outputs (emits) the generated incident signal to the probe 411, thereby irradiating the measurement target with the incident signal.

In step S104, the RF transmission/reception unit 412 receives the incident signal output in step S103 via a directional coupler (not illustrated), and performs AD conversion, and the like, on the received incident signal.

Furthermore, the RF transmission/reception unit 412 receives the reflected signal and the transmitted signal incident from the measurement target via the probe 411 by irradiating the measurement target with the incident signal, and performs AD conversion, and the like, on the received reflected signal and transmitted signal.

The RF transmission/reception unit 412 supplies AD data of the channel to be processed obtained by AD conversion, and the like, on the incident signal, the reflected signal, and the transmitted signal to the S parameter calculation unit 423. As a result, the AD data corresponding to one time of irradiation of the incident signal of the channel to be processed, more specifically, corresponding to one predetermined time section is obtained.

In step S105, the S parameter calculation unit 423 calculates the S parameter on the basis of the AD data supplied from the RF transmission/reception unit 412, and supplies the S parameter to the averaging processing unit 424.

In step S106, the transmission/reception control unit 421 determines whether or not the processing of calculating the S parameter has been performed the average number of times read from the average number of times storage unit 422.

In a case where it is determined in step S106 that the processing has not been performed the average number of times, thereafter, the processing returns to step S103, and the above-described processing is repeatedly performed.

In this case, irradiation of the incident signal by the RF transmission/reception unit 412 is continuously performed, and the S parameter calculation unit 423 calculates the S parameter on the basis of the AD data for each of time sections sequentially supplied from the RF transmission/reception unit 412.

On the other hand, in a case where it is determined in step S106 that the processing has been performed the average number of times, the transmission/reception control unit 421 instructs the S parameter calculation unit 423 to end the calculation of the S parameter, and thereafter, the processing of step S107 is performed.

In step S107, the averaging processing unit 424 calculates the average S parameter on the basis of the S parameters of the channel to be processed supplied from the S parameter calculation unit 423 on the basis of the average number of times of the channel to be processed supplied from the transmission/reception control unit 421. For example, in step S107, the average value of the S parameters of the number corresponding to the average number of times is calculated as the average S parameter.

The averaging processing unit 424 supplies the calculated average S parameter of the channel to be processed to the result holding unit 425 and causes the result holding unit 425 to hold (store) the average S parameter.

In step S108, the transmission/reception control unit 421 determines whether or not the processing has been performed on all the channels.

For example, in a case where the average S parameter is calculated for all the channels for which measurement is to be performed, which are set as channels to be processed, it is determined that processing has been performed on all the channels.

In a case where it is determined in step S108 that the processing has not been performed on all the channels, thereafter, the processing returns to step S101, and the above-described processing is repeatedly performed. In other words, a new channel is set as the channel to be processed, and the average S parameter of the channel is calculated.

On the other hand, in a case where it is determined in step S108 that the processing has been performed on all the channels, the measurement processing ends.

As described above, the measurement apparatus 401 performs processing of calculating the average S parameter over the average number of times set for each channel. This makes it possible to improve measurement accuracy while suppressing increase in a measurement period and power consumption.

Note that the methods from the first method for setting the average number of times to the third method for setting the average number of times have been described above as specific examples of the method for setting the average number of times for each frequency (channel).

However, the present technology is not limited thereto, and for example, the average number of times may be set in units of the frequency (GHz) of the incident signal, or a range and an interval of the channel serving as the channel region may be arbitrarily changed.

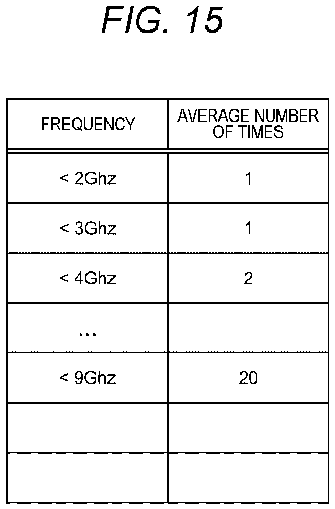

Specifically, in a case where the average number of times is set in units of the frequency, for example, a table of the average number of times for each frequency indicated in Fig. 15 may be stored in the average number of times storage unit 422 as the average number of times information.

In the example of Fig. 15, in the table of the average number of times for each frequency, the frequency, more specifically, the frequency band and the average number of times are stored in association with each other. In other words, the table of the average number of times for each frequency is information indicating an average number of times for each frequency band for all frequencies that can be taken as the frequency of the RF signal (incident signal) with which the measurement target is irradiated.

For example, the average number of times "1" is associated with "< 2 GHz" in the field of the frequency. Thus, for example, the average number of times "1" is used for a channel having a frequency of less than 2 GHz.

Furthermore, for example, the average number of times "1" is associated with "< 3 GHz" in the field of the frequency, and thus, it can be seen that the average number of times "1" is used for a channel having a frequency equal to or higher than 2 GHz and less than 3 GHz.

In addition, for example, even in a case where the average number of times is set for each frequency band, that is, for each region including one or a plurality of frequencies, as in the example described with reference to Fig. 12, an exceptional frequency in the region and the average number of times at the exceptional frequency may be separately set. Furthermore, in a case where the average number of times is set for each frequency band, an interval (the number of frequencies belonging to the region) and a range of the frequency band may be arbitrarily changed (settable).

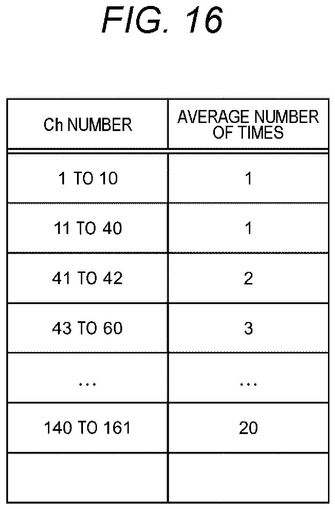

As another example, in a case where the range or the interval (number) of the channel to be the channel region can be arbitrarily changed, for example, the table of the average number of times for each channel region indicated in Fig. 16 may be stored in the average number of times storage unit 422 as the average number of times information.

In the example of Fig. 16, in a similar manner to the example illustrated in Fig. 11, "Ch number" indicating a channel belonging to a channel region and "average number of times" of each channel belonging to the channel region are stored in association with each other.

However, in the example of Fig. 16, the number of channels constituting the channel region and a range from the first channel to the last channel constituting the channel region, that is, the channels constituting the channel region can be arbitrarily changed (settable) for each channel region.

Specifically, for example, in the example of Fig. 11, the number of channels constituting each channel region is 20, which is a fixed value.

On the other hand, in the example of Fig. 16, the number of channels constituting each channel region is an arbitrary number, such as a channel region including 10 channels and a channel region including 30 channels.

Furthermore, for example, in the example of Fig. 16, it can be seen that a start position (channel with the smallest channel number) and an end position (channel with the largest channel number) of the channel region can also be arbitrarily set, for example, a fourth channel region is started from a channel with the channel number "43".

In addition, the average number of times information such as the table of the average number of times for each channel region stored in the average number of times storage unit 422 of the measurement apparatus 401 may be updated by an upper host of the measurement apparatus 401.

In such a case, for example, a block corresponding to the microcomputer 63 or the main CPU 52 of Fig. 2 functioning as a host supplies new average number of times information to the signal processing unit 413, and causes the average number of times information to be stored in the average number of times storage unit 422. As a result, the new average number of times information is stored in the average number of times storage unit 422, or the average number of times information already stored in the average number of times storage unit 422 is replaced with the newly supplied average number of times information, and the average number of times information is updated.

In addition, the host that updates the average number of times information may perform updating with reference to a cloud, or the like. For example, in a case where the measurement apparatus 401 is applied to a soil moisture sensor, a predicted moisture amount may be calculated from weather or time, and the average number of times information according to the predicted moisture amount may be set via a cloud.

According to the measurement apparatus 401 as described above, for example, if an average number of times with a good balance between measurement accuracy and power consumption is set in advance for each frequency, the number of times of transmission (output) of the incident signal can be set to 1/2 or less as compared with a case where the average number of times is fixed in all bands.

Furthermore, as a method for determining the average number of times, for example, in the measurement apparatus, a method in which the incident signal is output (transmitted) immediately before measurement is performed, and the average number of times is obtained by calculation from the reflected signal, or the like, obtained according to the output can be considered. However, in this method, in order to sequentially calculate the average number of times, it is necessary to output an incident signal for calculation, that is, to transmit a radio wave, separately from the incident signal for actual measurement.

On the other hand, in the measurement apparatus 401, the average number of times for each frequency is set in advance, and thus, radio wave transmission for calculation (output of the incident signal) is unnecessary, and measurement accuracy can be improved more easily and with lower power consumption and in a shorter measurement period.

Furthermore, there may be a case where transmission power of the incident signal needs to be reduced only at a specific frequency (channel) due to a wireless legal test measure, or the like. Even in such a case, the measurement apparatus 401 can individually set the average number of times for the specific frequency (channel), so that it is possible to increase the average number of times only for the specific frequency and improve the measurement accuracy.

Note that embodiments of the present technology are not limited to the above-described embodiments, and various changes can be made without departing from the gist of the present technology.

For example, in the present technology, two or more of the first to sixth embodiments described above can be appropriately combined.

Furthermore, for example, the present technology can be configured as cloud computing in which a plurality of apparatuses shares a single function and jointly performs processing over a network.

Furthermore, each step described in the flowchart described above may be executed by a single apparatus, or may be executed by a plurality of apparatuses in a shared manner.

Moreover, in a case where a single step includes a plurality of kinds of processing, the plurality of kinds of processing included in the single step can be performed by a single apparatus or performed by a plurality of apparatuses in a shared manner.

Moreover, the present technology may also have following configurations.
(1) A measurement apparatus including:
   a circuit portion that is arranged on one surface of a sensor substrate and measures an S parameter; and
   a sensor electrode that is arranged on the other surface of the sensor substrate and irradiates a measurement target with a signal for measuring the S parameter output from the circuit portion, in which
   a distance between the circuit portion and the sensor electrode is equal to or less than a predetermined distance in plan view, and
   a terminal that outputs the signal of the circuit portion is directly connected to the sensor substrate.
(2) The measurement apparatus according to (1), in which the circuit portion and the sensor electrode are arranged such that the circuit portion overlaps with the sensor electrode in plan view.
(3) The measurement apparatus according to (1), further including a device arranged on the one surface of the sensor substrate and connected between the circuit portion and the sensor electrode, in which
   the circuit portion outputs the signal to the sensor electrode via the device, and
   the device and the sensor electrode are arranged such that the device overlaps with the sensor electrode in plan view.
(4) The measurement apparatus according to any one of (1) to (3), in which the circuit portion is a VNA integrated circuit.
(5) The measurement apparatus according to any one of (1) to (4), in which an in-substrate wiring that is directly connected to the terminal of the circuit portion and connects the terminal and the sensor electrode is provided in the sensor substrate.
(6) The measurement apparatus according to (5), in which the in-substrate wiring is a linear wiring penetrating the sensor substrate.
(7) The measurement apparatus according to any one of (1) to (6), further including a contact detection sensor that detects a contact state of the sensor electrode with the measurement target.
(8) The measurement apparatus according to (7), in which the contact detection sensor detects the contact state by detecting a capacitance value or a resistance value of the sensor electrode.
(9) The measurement apparatus according to (7), in which the contact detection sensor detects the contact state by detecting a capacitance value or a resistance value of an electrode portion arranged in the vicinity of the sensor electrode on the other surface of the sensor substrate.
(10) The measurement apparatus according to any one of (7) to (9), further including a control unit that calculates a reliability parameter of biological information of the measurement target calculated from the S parameter on the basis of a detection result by the contact detection sensor.
(11) The measurement apparatus according to any one of (7) to (9), further including a control unit that determines whether or not to measure the S parameter on the basis of a detection result by the contact detection sensor.
(12) The measurement apparatus according to any one of (1) to (6), further including a control unit that determines a contact state of the sensor electrode with the measurement target on the basis of the S parameter.
(13) The measurement apparatus according to any one of (1) to (12), further including a detection unit that is provided in the vicinity of the circuit portion and detects a temperature of the measurement target,
   in which the circuit portion calculates the S parameter on the basis of the signal with which the measurement target is irradiated, a signal received from the measurement target in response to irradiation of the signal, and the temperature.
(14) The measurement apparatus according to (13), further including a biological information calculation unit that calculates biological information of the measurement target on the basis of the temperature and the S parameter.
(15) The measurement apparatus according to (14), further including a control unit that calculates a reliability parameter of the biological information on the basis of the temperature.
(16) The measurement apparatus according to (1) or (2), in which
   the sensor substrate includes a first substrate portion on which the circuit portion is provided and a second substrate portion on which the sensor electrode is provided, and
   a thermally conductive material is arranged between a surface of the circuit portion and a surface of the second substrate portion.
(17) The measurement apparatus according to (16), in which the thermally conductive material is a TIM.
(18) The measurement apparatus according to (16) or (17), in which a copper foil is provided at a portion in contact with the thermally conductive material on a surface of the second substrate portion.
(19) The measurement apparatus according to any one of (1) to (12), further including a body motion detection unit that detects a body motion state of a wearer of the measurement apparatus, in which
   the circuit portion calculates the S parameter on the basis of the signal emitted to the measurement target, a signal received from the measurement target in response to irradiation of the signal, and a detection result of the body motion state.
(20) The measurement apparatus according to (19), further including a control unit that calculates a reliability parameter of the biological information of the measurement target calculated from the S parameter on the basis of a detection result of the body motion state.
(21) The measurement apparatus according to any one of (1) to (12), further including a biological information calculation unit that calculates biological information of the measurement target on the basis of the S parameter.
(22) The measurement apparatus according to (21), in which the biological information is a glucose level.
(23) The measurement apparatus according to any one of (1) to (12), in which the circuit portion includes
   an S parameter calculation unit that calculates the S parameter of a predetermined frequency with which the measurement target is irradiated on the basis of the signal of the frequency and a signal received from the measurement target in response to irradiation of the signal of the frequency,
   an average number of times storage unit that stores average number of times information indicating an average number of times of the S parameter set in advance at the frequency, and
   an averaging processing unit that calculates an average value of the S parameters of the number corresponding to the average number of times indicated by the average number of times information for each frequency.
(24) The measurement apparatus according to (23), in which the average number of times information is information indicating the average number of times for each frequency of the signal with which the measurement target is irradiated or for each channel corresponding to the frequency.
(25) The measurement apparatus according to (23), in which the average number of times information is information indicating the average number of times for each region including one or a plurality of the frequencies of the signal with which the measurement target is irradiated or including one or a plurality of channels corresponding to the frequencies.
(26) The measurement apparatus according to (25), in which the number of the frequencies or the channels belonging to the region, or the range of the frequencies or the channels belonging to the region can be arbitrarily set for each region.
(27) The measurement apparatus according to (23), in which the average number of times information is information indicating the average number of times for each region including one or a plurality of the frequencies of the signal with which the measurement target is irradiated or including one or a plurality of channels corresponding to the frequencies, and the average number of times of the frequencies or the channels that are exceptional among the frequencies or the channels belonging to the region.
(28) The measurement apparatus according to any one of (23) to (27), in which the average number of times information stored in the average number of times storage unit is updatable.
(29) The measurement apparatus according to any one of (1) to (28), in which the measurement target is a living body.

### REFERENCE SIGNS LIST

- 11: Smartwatch
- 51: Glucose sensing module
- 52: Main CPU
- 61: Sensor substrate
- 62: VNA chip
- 63: Microcomputer
- 64: Temperature sensor
- 65: Flash memory
- 91: Sensor electrode
- 141: Device
- 171: Sensor
- 401: Measurement apparatus
- 411: Probe
- 412: RF transmission/reception unit
- 413: Signal processing unit
- 421: Transmission/reception control unit
- 422: Average number of times storage unit
- 423: S parameter calculation unit
- 424: Averaging processing unit

## Claims

1. A measurement apparatus comprising:
a circuit portion that is arranged on one surface of a sensor substrate and measures an S parameter; and
a sensor electrode that is arranged on the other surface of the sensor substrate and irradiates a measurement target with a signal for measuring the S parameter output from the circuit portion, wherein
a distance between the circuit portion and the sensor electrode is equal to or less than a predetermined distance in plan view, and
a terminal that outputs the signal of the circuit portion is directly connected to the sensor substrate.

2. The measurement apparatus according to claim 1, wherein the circuit portion and the sensor electrode are arranged such that the circuit portion overlaps with the sensor electrode in plan view.

3. The measurement apparatus according to claim 1, further comprising a device arranged on the one surface of the sensor substrate and connected between the circuit portion and the sensor electrode, wherein
the circuit portion outputs the signal to the sensor electrode via the device, and
the device and the sensor electrode are arranged such that the device overlaps with the sensor electrode in plan view.

4. The measurement apparatus according to claim 1, wherein the circuit portion is a VNA integrated circuit.

5. The measurement apparatus according to claim 1, wherein
an in-substrate wiring that is directly connected to the terminal of the circuit portion and connects the terminal and the sensor electrode is provided in the sensor substrate.

6. The measurement apparatus according to claim 5, wherein the in-substrate wiring is a linear wiring penetrating the sensor substrate.

7. The measurement apparatus according to claim 1, further comprising a contact detection sensor that detects a contact state of the sensor electrode with the measurement target.

8. The measurement apparatus according to claim 7, wherein the contact detection sensor detects the contact state by detecting a capacitance value or a resistance value of the sensor electrode.

9. The measurement apparatus according to claim 7, wherein the contact detection sensor detects the contact state by detecting a capacitance value or a resistance value of an electrode portion arranged in the vicinity of the sensor electrode on the other surface of the sensor substrate.

10. The measurement apparatus according to claim 7, further comprising a control unit that calculates a reliability parameter of biological information of the measurement target calculated from the S parameter on a basis of a detection result by the contact detection sensor.

11. The measurement apparatus according to claim 7, further comprising a control unit that determines whether or not to measure the S parameter on a basis of a detection result by the contact detection sensor.

12. The measurement apparatus according to claim 1, further comprising a control unit that determines a contact state of the sensor electrode with the measurement target on a basis of the S parameter.

13. The measurement apparatus according to claim 1, further comprising a detection unit that is provided in the vicinity of the circuit portion and detects a temperature of the measurement target,
wherein the circuit portion calculates the S parameter on a basis of the signal with which the measurement target is irradiated, a signal received from the measurement target in response to irradiation of the signal, and the temperature.

14. The measurement apparatus according to claim 13, further comprising a biological information calculation unit that calculates biological information of the measurement target on a basis of the temperature and the S parameter.

15. The measurement apparatus according to claim 14, further comprising a control unit that calculates a reliability parameter of the biological information on a basis of the temperature.

16. The measurement apparatus according to claim 1, wherein the sensor substrate includes a first substrate portion on which the circuit portion is provided and a second substrate portion on which the sensor electrode is provided, and
a thermally conductive material is arranged between a surface of the circuit portion and a surface of the second substrate portion.

17. The measurement apparatus according to claim 16, wherein the thermally conductive material is a TIM.

18. The measurement apparatus according to claim 16, wherein a copper foil is provided at a portion in contact with the thermally conductive material on a surface of the second substrate portion.

19. The measurement apparatus according to claim 1, further comprising a body motion detection unit that detects a body motion state of a wearer of the measurement apparatus, wherein
the circuit portion calculates the S parameter on a basis of the signal emitted to the measurement target, a signal received from the measurement target in response to irradiation of the signal, and a detection result of the body motion state.

20. The measurement apparatus according to claim 19, further comprising a control unit that calculates a reliability parameter of the biological information of the measurement target calculated from the S parameter on a basis of a detection result of the body motion state.

21. The measurement apparatus according to claim 1, further comprising a biological information calculation unit that calculates biological information of the measurement target on a basis of the S parameter.

22. The measurement apparatus according to claim 21, wherein the biological information is a glucose level.

23. The measurement apparatus according to claim 1, wherein the circuit portion includes:
an S parameter calculation unit that calculates the S parameter of a predetermined frequency with which the measurement target is irradiated on a basis of the signal of the frequency and a signal received from the measurement target in response to irradiation of the signal of the frequency;
an average number of times storage unit that stores average number of times information indicating an average number of times of the S parameter at the frequency set in advance; and
an averaging processing unit that calculates an average value of the S parameters of the number corresponding to the average number of times indicated by the average number of times information for each frequency.

24. The measurement apparatus according to claim 23, wherein the average number of times information is information indicating the average number of times for each frequency of the signal with which the measurement target is irradiated or for each channel corresponding to the frequency.

25. The measurement apparatus according to claim 23, wherein the average number of times information is information indicating the average number of times for each region including one or a plurality of the frequencies of the signal with which the measurement target is irradiated or including one or a plurality of channels corresponding to the frequencies.

26. The measurement apparatus according to claim 25, wherein the number of the frequencies or the channels belonging to the region, or a range of the frequencies or the channels belonging to the region can be arbitrarily set for each region.

27. The measurement apparatus according to claim 23, wherein the average number of times information is information indicating the average number of times for each region including one or a plurality of the frequencies of the signal with which the measurement target is irradiated or including one or a plurality of channels corresponding to the frequencies, and the average number of times of the frequencies or the channels that are exceptional among the frequencies or the channels belonging to the region.

28. The measurement apparatus according to claim 23, wherein the average number of times information stored in the average number of times storage unit is updatable.

29. The measurement apparatus according to claim 1, wherein the measurement target is a living body.
